# EUROPEAN PATENT APPLICATION

(11) **EP 2 893 865 A1**
(43) Date of publication of application: **15.07.2015**
(21) Application number: 14761169.3
(22) Date of filing: 10.01.2014
(51) Int. Cl.: A61B 1/00, G02B 23/24

(54) **ENDOSCOPE**

(30) Priority: 05.03.2013 JP 2013043303
(71) Applicant: OLYMPUS CORPORATION, Shibuya-ku Tokyo 151-0072 (JP)
(72) Inventor: FUKUDA, Hiroyuki, Tokyo 151-0072 (JP); FUNAKOSHI, Yasuo, Tokyo 151-0072 (JP); SUGAYA, Kota, Tokyo 151-0072 (JP); TAJIMA, Kenji, Tokyo 151-0072 (JP); HOSHINO, Yuki, Tokyo 151-0072 (JP)
(74) Representative: Schicker, Silvia
(86) International application number: PCT/JP2014/050326
(87) International publication number: WO 2014/136470

(57) **Abstract**

An endoscope 1 includes: an operation portion 3 including a grasping portion 3A and a grasp-assistance face 35a on which a finger of an operator is to be disposed when the operator grasps the grasping portion 3A; an insertion portion 2 including on a distal end side thereof a bending portion 2b; at least one knob 5 that is provided on one side face of the operation portion 3 and that, by a rotational operation centering on a rotation axis 5a, causes the bending portion 2b to bend; at least two buttons including an air/water feeding button 6a and a suction button 6b that are disposed so as to protrude from a fluid control button arrangement portion 32 constituting an upper side of the operation portion 3, and that are arranged in parallel in a longitudinal axis direction of the operation portion 3, wherein switch root portions 6ar, 6br of the buttons 6a, 6b are disposed so as to be positioned between two intersection points P1, P2 at which an outermost diametrical path 5S of an up/down knob 5 that is projected onto a plane having a normal line along a rotation central axis and the fluid control button arrangement portion 32 intersect with each other.

## Description

### Technical Field

The present invention relates to an endoscope having an operation portion equipped with a bending operation knob that causes a bending portion provided in an insertion portion to perform a bending operation and a plurality of function switches that operate endoscope functions.

### Background Art

Endoscopes have been widely used in the medical field and industrial field in recent years. The available kinds of endoscopes include an optical fiber endoscope that uses image fibers as image transmitting means, and an electronic endoscope that contains an image pickup device in a distal end portion of an insertion portion.

In the case of an electronic endoscope, an endoscope observation image of a site to be observed that is picked up by an image pickup device is displayed on an observation screen, and observation or the like is performed. On the other hand, in the case of an optical fiber endoscope, observation is performed directly with the naked eye through an ocular portion, or a camera is attached to an ocular portion and an optical image transmitted to the ocular portion is displayed on an observation screen and observation or the like is performed.

An endoscope that is used in the medical field is configured to enable observation or the like to be performed by inserting a rigid insertion portion or a flexible insertion portion into the body.

Some endoscopes include a bending portion on a distal end side of the insertion portion. The bending portion is configured to bend in two directions, namely, upward and downward, or in four directions, namely, upward, downward, left and right. In an endoscope in which the bending portion is provided, observation or the like can be performed over a wide range by changing the observation direction of an observation optical system provided in a distal end portion by causing the bending portion to perform a bending operation.

A plurality of bending wires that correspond to bending directions are extended from the bending portion. A bending operation apparatus provided in the operation portion of the endoscope is connected to end portions of the extended bending wires. The bending operation apparatus is an apparatus that causes the bending portion to perform a bending operation. The bending portion is configured to perform a bending operation upon a bending wire being pulled or slackened by an operation of the bending operation apparatus.

The bending operation apparatus and endoscope function switches are provided in the operation portion of an electronic endoscope. Examples of the bending operation apparatus include a bending operation knob, a bending operation lever, and a joystick.

Fluid control buttons and electrical switches are provided as function switches. The fluid control buttons include an air/water feeding button and a suction button. The electrical switches include a release switch that generates a release signal when photographing, a freeze switch that generates a freeze signal, and an observation mode switching switch for issuing an instruction to switch an observation mode.

An operator that operates the endoscope uses the fingers with which they grasp the operation portion to operate the bending operation apparatus and the various function switches.

Japanese Patent Application Laid-Open Publication No. 2002-58629 discloses an electronic endoscope having excellent operability with which, when performing endoscope operations, the operations can be performed without placing an excessive burden on the operator. In the electronic endoscope discussed in the aforementioned Japanese Patent Application Laid-Open Publication No. 2002-58629, a bending operation knob, a suction button, an air/water feeding button and a control switch and the like are provided in an operation-portion operation region of the operation portion. A first hood portion is provided between the suction button and the air/water feeding button. A second hood portion is provided on the lower side of the air/water feeding button. The second hood portion forms a grasp-assistance portion with which the fingers of the operator which grasp the operation portion engage.

Further, a first hood portion and a second hood portion that are constructed by providing a step portion in an operation portion 3 are also disclosed in Fig. 3 of Japanese Patent Application Laid-Open Publication No. 2002-58629.

According to the above-described electronic endoscope, by placing their left hand on one side face and the other side face of the operation portion and on the lower side of the second hood portion, the operator can hold the operation portion in a stable state and perform an operation to insert the insertion portion into a body cavity. Further, by checking the first hood portion, the operator can prevent an erroneous operation of a button that is different to an intended button.

On the other hand, Japanese Patent Application Laid-Open Publication No. 2004-141331 discloses an endoscope operation apparatus that facilitates operations with respect to a conduit control operation member and various function switches that are disposed on an operation portion using fingers of one hand that grasps the operation portion. In this endoscope operation apparatus, a suction operation valve, an air/water feeding operation valve, a first function switch and a second function switch are disposed side by side on one side face of an operation portion constituted by a single case body, and operational upper faces of these switches and the like are disposed at approximately the same distance r from a rotational axis center Oa of an angle operation knob.

According to this configuration, an operation in which an operator grasps an operation portion shown in Fig. 1 and Fig. 2 of Japanese Patent Application Laid-Open Publication No. 2004-141331 from the rear side in the drawings and operates a left/right angle knob (corresponds to a bending operation knob of the present invention) and an upward/downward angle knob or the like using the thumb, and also operates the suction operation valve, the air/water feeding operation valve, the first function switch and the second function switch using the index finger and middle finger is made exceptionally easy. Further, the index finger reaches the suction operation valve, the first function switch and the second function switch without any strain. In addition, since the operation members are centered around the suction operation valve that has the highest height in a configuration such that the heights of the other operation members become progressively lower in sequence from the suction operation valve and, furthermore, the heights of the operation members are set in a circular arc shape, it is easy for the operator to distinguish the respective operation members. Further, the operator can hold the operation portion in a stable state by placing the cable on the crotch between the thumb and index finger of the left hand, placing the palm of the left hand on the one side face and the other side face of the operation portion, and placing the middle finger or ring finger of the left hand on a portion on the rear side of the operation portion discussed in Japanese Patent Application Laid-Open Publication No. 2004-141331 that corresponds to the second hood portion discussed in Japanese Patent Application Laid-Open Publication No. 2002-58629.

However, the endoscope operation apparatus discussed in Japanese Patent Application Laid-Open Publication No. 2004-141331 has a restriction in that, as shown in Fig. 1A, the respective operational upper faces provided on the operation portion side face are arranged at approximately the same distance from the rotational axis center of the angle operation knob, as shown by a chain double-dashed line. This restriction makes it difficult to set the height of the air/water feeding valve 21 to the height same as that of the suction operation valve 20, or set the heights of the switches to relatively high.

An operation that rotates a bending operation knob is not limited to an operation performed using the thumb of the left hand of the operator, and is also commonly performed using the middle finger or ring finger of the left hand. In addition, depending on the operator, the rotation operation may be performed with two fingers, namely, the thumb and, for example, ring finger of the left hand. By performing an operation to rotate the bending operation knob with two fingers, fine adjustment of the bending amount of the bending portion can be performed with ease. However, when the operator operates the bending operation knob with the middle finger or the ring finger, the operator has to operate the knob across the buttons arrayed on the operation portion side face. If the operator is a person with small hands, it is difficult to smoothly perform the rotational operation of the bending operation knob with the middle finger or the ring finger across the buttons. To address such a difficulty, a configuration for reducing the heights of the buttons or increasing the outer diameter of the knob can be considered. However, when the operator operates the bending operation knob with the middle finger or the ring finger across the buttons, there is a possibility of erroneous operation of the air/water feeding valve, etc.

Specifically, in a case where a knob 16 shown in Japanese Patent Application Laid-Open Publication No. 2004-141331 is, for example, as shown in Fig. 1B, pinched between the thumb and ring finger and rotated in the direction of an arrow Y1B (counterclockwise rotation) as indicated by a broken line, accompanying the rotational movement of the knob 16, a state is entered in which the ring finger passes across a button (designated by reference numeral 20) to operate the knob 16.

Under such a state, the operator with small hands possibly cannot continue the rotation operation of the knob 16 by the ring finger contacting the air/water feeding valve during the rotation operation, or the ring finger possibly gets away from the knob 16.

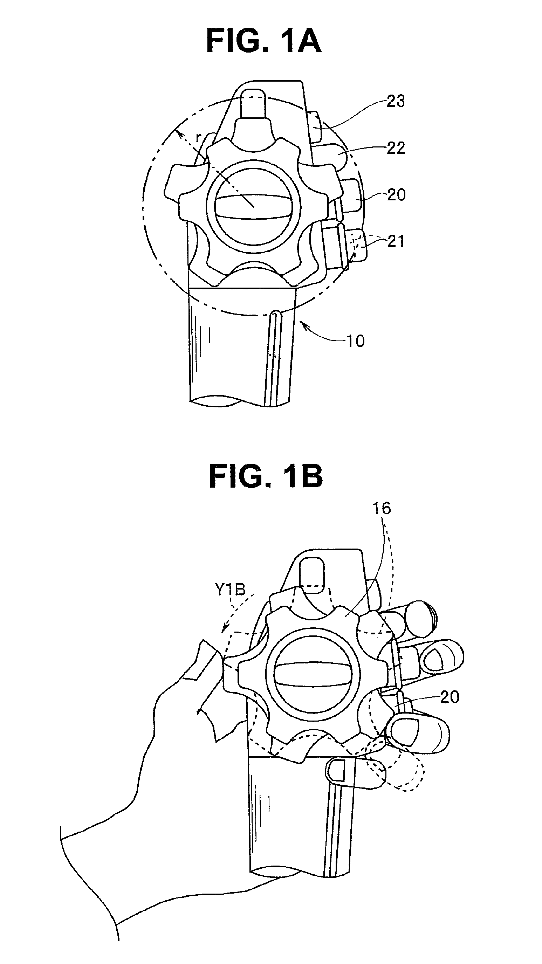

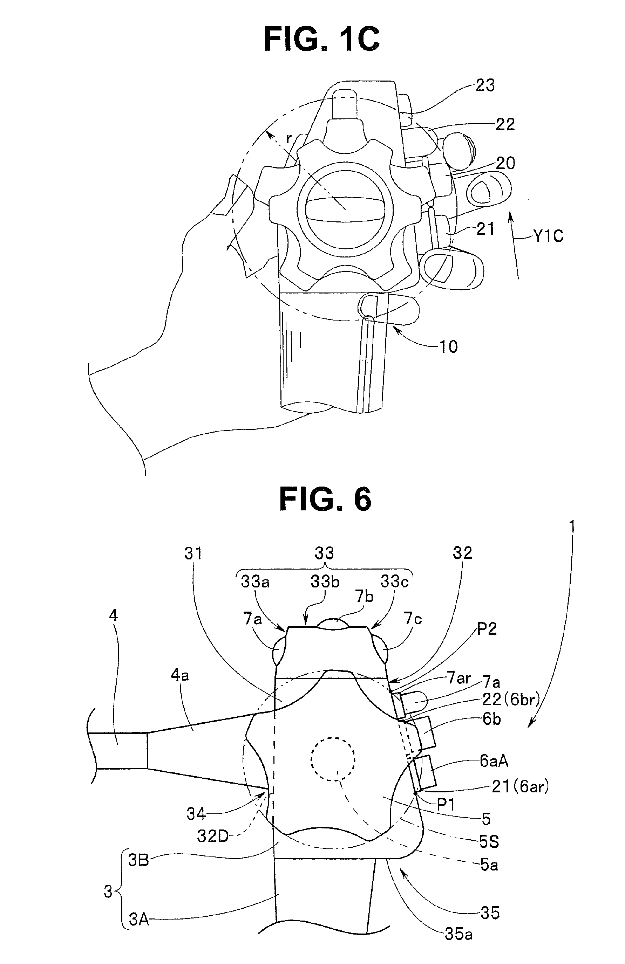

As shown in Fig. 1C, by moving the air/water feeding valve 21, the suction operation valve 20, the first function switch 22 and the second function switch 23 in the direction of an arrow Y1C on the operation portion side face, an operation in which the ring finger passes across a button no longer arises. However, when these switches are simply displaced, it is difficult to arrange the operational upper faces of the switches, etc., provided on the operation portion side face at approximately the same distance r from the above-described rotational axis center. In order to arrange the operational upper faces within approximately the same distance from the rotational axis center, it is necessary to change the position of the rotational axis center and set a new distance R.

The present invention has been achieved in view of the above-described circumstances, and an object of the present invention is to provide an endoscope provided with an endoscope operation portion that enables rotation operation of a bending operation knob to be surely and smoothly performed with the thumb and another finger of a hand grasping the operation portion, with the operation portion being held in a stably state with one hand.

### Disclosure of Invention

### Means for Solving the Problem

An endoscope according to one aspect of the present invention includes: an operation portion including a grasping portion which is grasped by an operator and a grasp-assistance face on which the operator performs operation and a finger of the operator is to be disposed when the operator grasps the grasping portion; an insertion portion extended from a distal end side of the operation portion, the insertion portion including on a distal end side thereof a bending portion; at least one bending operation knob that is provided on one side face of the operation portion and that, by a rotational operation centering on a rotation axis, causes the bending portion to bend; and at least two function switches that are provided so as to protrude from a side face on one side that configures a side in one direction of the one side face of the operation portion, the at least two function switches being disposed along a longitudinal axis of the operation portion, wherein switch root portions of the function switches are disposed so as to be positioned between two intersection points at which an outermost diametrical path formed by an outermost circumferential portion of the bending operation knob that is projected onto a plane having a normal line along a rotation central axis of the bending operation knob and the side face on one side intersect with each other.

### Brief Description of the Drawings

Fig. 1A is a diagram illustrating a configuration example in which switches having the same heights are arranged according to the endoscope discussed in Patent Literature 2.
Fig. 1B is a diagram illustrating a state in which a knob of the endoscope discussed in Patent Literature 2 is rotationally operated using the thumb and ring finger.
Fig. 1C is a diagram illustrating a configuration example in which an operation in which a finger passes across a button no longer arises with respect to the endoscope discussed in Patent Literature 2.
Fig. 2 to Fig. 6 relate to one embodiment of the present invention, of which Fig. 2 is a diagram illustrating an endoscope of the present invention.
Fig. 3A is a diagram that illustrates the configuration of an operation portion body of an operation portion of the endoscope, and also illustrates a relation between an outermost diametrical path that is projected onto a plane having a normal line along a rotation central axis of a bending operation knob and a side face on one side.
Fig. 3B is a diagram illustrating an example of grasping the operation portion of the endoscope.
Fig. 4 is a diagram illustrating a state in which an up/down knob of the endoscope is pinched between a thumb and a ring finger and is being subjected to a rotational operation.
Fig. 5 is a diagram illustrating a configuration in which function switches having different heights are arrayed on a fluid control button arrangement portion of the operation portion body.
Fig. 6 is a diagram illustrating a configuration in which three function switches are arrayed between a first intersection point P1 and a second intersection point P2 that are formed between an outermost diametrical path and an upper-side side face.

### Best Mode for Carrying Out the Invention

An embodiment of the present invention is described hereunder with reference to the accompanying drawings.

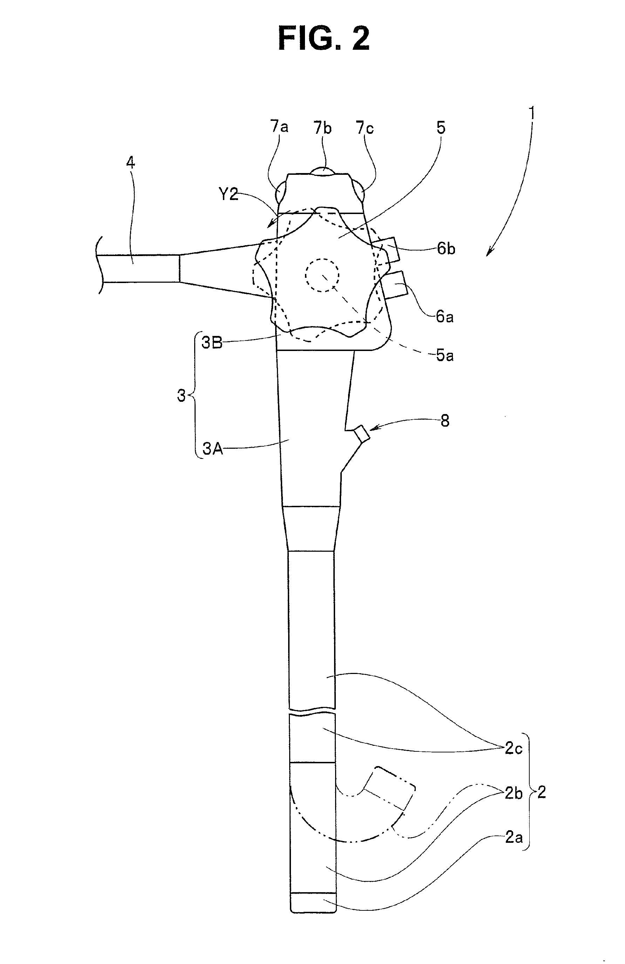

As shown in Fig. 2, an endoscope 1 mainly includes an insertion portion 2, an operation portion 3 and a universal cord 4.

The insertion portion 2 is inserted into a duct in the body such as the stomach or the large intestine. The insertion portion 2 includes a rigid distal end portion 2a, a bending portion 2b, and a flexible tube portion 2c having flexibility that are connected in series in that order from the distal end side. The bending portion 2b, for example, is configured to be bendable in the upward and downward directions.

An image pickup device (not illustrated in the drawings) is provided as observation means (not illustrated in the drawings) in the distal end portion 2a of the insertion portion 2. A nozzle (not illustrated in the drawings) and a treatment instrument channel opening (not illustrated in the drawings) from which a treatment instrument is led out and the like are also provided in the distal end portion 2a.

The operation portion 3 is provided on a proximal end side of the insertion portion 2. The operation portion 3 includes a grasping portion 3A and an operation portion body 3B in that order from the distal end side. The universal cord 4 extends from a side portion of the operation portion body 3B. An endoscope connector (not illustrated in the drawings) is provided at a proximal end portion of the universal cord 4. The endoscope connector is electrically connected to an external apparatus that includes a light source and a camera control unit and the like.

An up/down knob 5 that is a bending operation apparatus and a plurality of function switches are provided on the operation portion body 3B. The function switches are fluid control buttons and electrical switches 7. The fluid control buttons are, for example, an air/water feeding button 6a and a suction button 6b. The electrical switches 7 are, for example, an observation mode switching switch 7a, a release switch 7b and a freeze switch 7c.

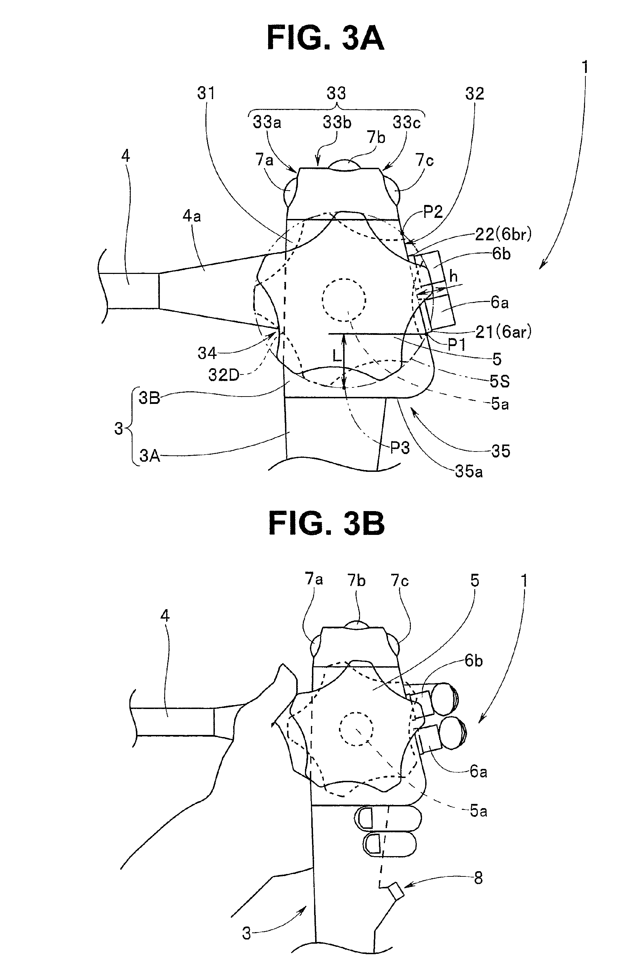

As shown in Fig. 3A, a bending operation apparatus arrangement portion 31, a fluid control button arrangement portion 32, and an electrical switch arrangement portion 33 are provided in the operation portion body 3B. Reference numeral 34 denotes a first grasp-assistance portion. Reference numeral 35 denotes a second grasp-assistance portion.

The bending operation apparatus arrangement portion 31 is one side face of the operation portion body 3B. The up/down knob 5 for performing an operation to bend the bending portion 2b is provided on the bending operation apparatus arrangement portion 31. The up/down knob 5 is configured to rotate clockwise and counterclockwise around a rotating shaft portion 5a.

If an operator rotates the up/down knob 5 in, for example, the counterclockwise direction that is the direction indicated by an arrow Y2 in Fig. 2 from the state indicated by a solid line, the bending portion 2b performs a bending operation in the upward direction that is one direction as shown by a chain double-dashed line in Fig. 2. On the other hand, if the operator rotates the up/down knob 5 in the clockwise direction that is the opposite direction to the aforementioned direction, the bending portion 2b performs a bending operation in the downward direction that is the other direction.

Note that, in the present embodiment, a center position of the rotating shaft portion 5a is set at a predetermined position within the one side face that is the bending operation apparatus arrangement portion 31.

As shown in Fig. 3A, the fluid control button arrangement portion 32 is a side in one direction (hereunder, referred to as "upper-side side face") of the one side face of the operation portion body 3B. In the fluid control button arrangement portion 32, an air/water feeding cylinder 21 and a suction cylinder 22 are disposed along a longitudinal axis of the operation portion 3.

Note that, the air/water feeding cylinder 21 and the suction cylinder 22, for example, may also be arrayed in parallel with the longitudinal axis of the operation portion 3. The air/water feeding button 6a is detachably mounted in the air/water feeding cylinder 21. The suction button 6b is detachably mounted in the suction cylinder 22.

The suction button 6b is slidably mounted with respect to the suction cylinder 22. Upon the operator performing an operation to switch the position of the button 6b from the natural state thereof to a predetermined position, a state is obtained in which dirt or the like is sucked from the treatment instrument channel opening.

The air/water feeding button 6a is slidably mounted with respect to the air/water feeding cylinder 21. Upon the operator gradually switching the position of the button 6a from the natural state thereof to a predetermined position, a state is obtained in which a gas such as air is ejected from the nozzle or in which a liquid such as water is ejected from the nozzle or the like.

In the present embodiment, a protruding height from the upper-side side face of the air/water feeding button 6a mounted in the air/water feeding cylinder 21 to the upper face of the cylinder and a protruding height from the upper-side side face of the suction button 6b mounted in the suction cylinder 22 to the upper face of the cylinder are set to the same height.

The electrical switch arrangement portion 33 is a proximal-end-side portion of the operation portion body 3B. The electrical switch arrangement portion 33 includes, for example, a first switch arrangement face 33a, a second switch arrangement face 33b, and a third switch arrangement face 33c. For example, an observation mode switching switch 7a is arranged on the first switch arrangement face 33a. For example, a release switch 7b is arranged on the second switch arrangement face 33b. For example, a freeze switch 7c is arranged on the third switch arrangement face 33c. The arrangement positions of the respective switches 7a, 7b, and 7c is not limited to the aforementioned arrangement faces 33a, 33b and 33c.

The observation mode switching switch 7a is a switch for issuing an instruction to switch the observation mode. The release switch 7b is a switch that generates a release signal when photographing. The freeze switch 7c is a switch that generates a freeze signal.

The first grasp-assistance portion 34 is constituted by a root portion of a bend preventer 4a that is included in the universal cord 4, and a lower-side side face 32D that is an opposite face to the upper-side side face of the operation portion body 3B. As shown in Fig. 3B, the crotch between the thumb and index finger of the left hand with which the operator grasps the operation portion 3, as well as the peripheral region of the crotch, are disposed on the first grasp-assistance portion 34.

As shown in Fig. 3A, the second grasp-assistance portion 35 is a step portion that is provided on a distal end side of the upper-side side face of the operation portion 3. The second grasp-assistance portion 35 has a grasp-assistance face 35a. As shown in Fig. 3B, a finger other than the thumb and index finger of the left hand with which the operator grasps the operation portion 3, for example, the ring finger, is disposed on the grasp-assistance face 35a.

When the operator grasps the operation portion 3 with their left hand, the vicinity of the crotch between the thumb and index finger of the left hand is disposed on the first grasp-assistance portion 34, and, for example, the ring finger of the left hand is disposed on the grasp-assistance face 35a. With the aforementioned fingers disposed in this state, the palm of the left hand is disposed on the grasping portion 3A of the operation portion 3. With the left hand disposed in this state, the operator can hold the operation portion 3 in a stable state.

Note that reference numeral 8 denotes a treatment instrument insertion port. A treatment instrument such as a grasping forceps is inserted into a treatment instrument channel (not illustrated in the drawings) from the treatment instrument insertion port 8, and is led out from the treatment instrument channel opening.

The air/water feeding cylinder 21 and the suction cylinder 22 that are illustrated in Fig. 3A are previously embedded in the operation portion body 3B. One end portion of the air/water feeding cylinder 21 and one end portion of the suction cylinder 22 are exposed to the outer side of the upper-side side face. The exposed one end portion of the air/water feeding cylinder 21 constitutes a root portion 6ar of the air/water feeding button 6a. The exposed one end portion of the suction cylinder 22 constitutes a root portion 6br of the suction button 6b.

In the present embodiment, a cross-sectional shape that is perpendicular to the longitudinal axis of the air/water feeding cylinder 21 is an annular shape. A cross-sectional shape that is perpendicular to the longitudinal axis of the suction cylinder 22 is also an annular shape.

The root portion 6ar of the air/water feeding button 6a and the root portion 6br of the suction button 6b are disposed between a first intersection point P1 and a second intersection point P2. The first intersection point P1 and the second intersection point P2 are intersection points between an outermost diametrical path 5S, which is indicated by a chain double-dashed line, that the outermost circumferential portion of the up/down knob 5 forms, and the upper-side side face that is the fluid control button arrangement portion 32. The intersection points P1 and P2 are two intersecting points formed when the outermost diametrical path 5S and the upper-side side face are projected onto a plane having a normal line along the central axis of the rotating shaft portion 5a of the up/down knob 5.

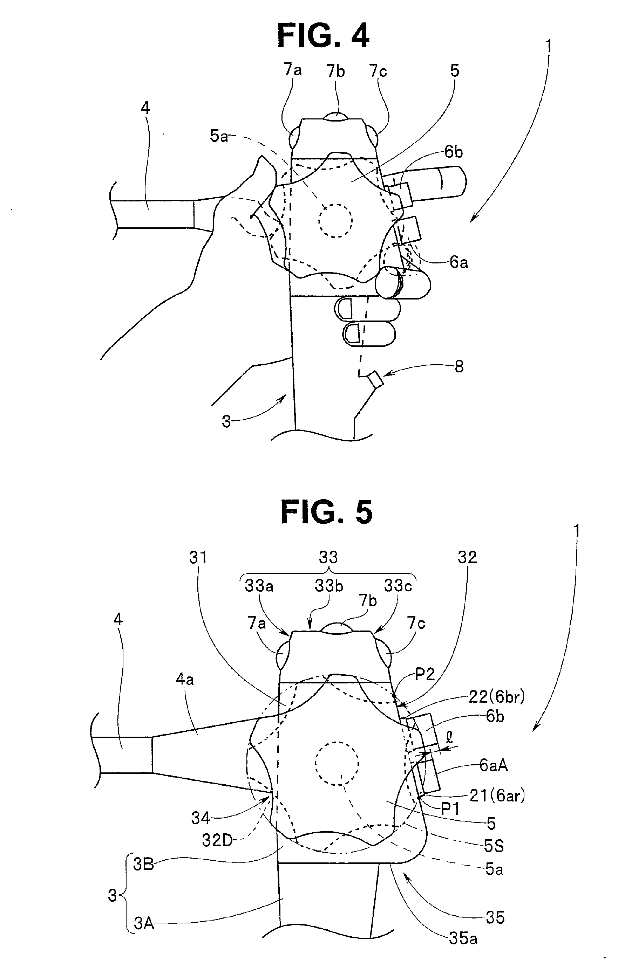

According to the above-described configuration, as shown by a solid line in Fig. 4, the operator, for example, pinches the up/down knob 5 between the thumb and middle finger. Thereafter, the operator starts to rotate the knob 5. When the up/down knob 5 is rotated, the positions of the thumb and middle finger change accompanying the rotation.

Subsequently, when the operator rotates the up/down knob 5, for example, until entering the state indicated by a dashed line, the operator holds the knob 5 with the ring finger and changes the position at which the thumb contacts the up/down knob 5. Next, the operator holds the knob 5 with the thumb, and changes the position at which the middle finger contacts the up/down knob 5. Thereafter, the operator repeatedly rotates the up/down knob 5 in the same manner as described above. Thus, the operator can place the bending portion 2b in a state of maximum bending with respect to the upward direction.

At this time, an operation in which the operator pinches the up/down knob 5 with the middle finger by passing the middle finger across the air/water feeding button 6a does not arise.

In the present embodiment, the outermost diametrical path 5S shown in Fig. 3 is set based on three points, namely, a distalmost position of an outermost diametrical path P3 that is set at a predetermined position within the one side face that is the bending operation apparatus arrangement portion 31, the first intersection point P1 and the second intersection point P2. By setting the outermost diametrical path 5S based on these points P1, P2 and P3, rotation of the up/down knob 5 can be continuously performed by appropriately changing the positions at which the up/down knob 5 is pinched by the thumb and middle finger, without performing a knob operation in which the middle finger or the like passes across a button.

Note that, the distalmost position of an outermost diametrical path P3 is provided at a location that is separated by a distance L in the distal end direction of the longitudinal axis of the operation portion 3 from the first intersection point P1.

In the configuration in which the up/down knob 5 as well as the air/water feeding button 6a and suction button 6b are provided as two function switches on the operation portion 3 having the grasp-assistance face 35a in this manner, the root portion 6ar of the air/water feeding button 6a and the root portion 6br of the suction button 6b are disposed between the first intersection point P1 and the second intersection point P2 that are intersection points between the outermost diametrical path 5S and the fluid control button arrangement portion 32. Consequently, an operation to continuously rotate the up/down knob 5 can be performed while pinching the up/down knob 5 between the thumb and middle finger or between the thumb and ring finger.

Further, the protruding height from the upper-side side face of the air/water feeding button 6a and the protruding height from the upper-side side face of the suction button 6b can be set to a same height h.

Note that, in the above described embodiment, a configuration is adopted in which the protruding height of the air/water feeding button 6a and the protruding height of the suction button 6b are set to the same height h. However, as shown in Fig. 5, a configuration may also be adopted in which a protruding height of an air/water feeding button 6aA is set so as to be lower than the protruding height of the suction button 6b by a predetermined dimension 1.

As a result, without visually checking the positions of the buttons 6aA and 6b of the operation portion 3, the operator can, for example, move the middle finger that operates the up/down knob 5 to confirm the difference in the heights of the buttons, and thereby easily distinguish the respective buttons.

In the above described embodiment, the air/water feeding button 6a and the suction button 6b are arranged as two function switches. However, the function switches provided on the upper-side side face are not limited to the air/water feeding button 6a and the suction button 6b, and may also be other function switches.

In addition, in the above-described embodiment, two function switches are arranged on the upper-side side face. However, as shown in Fig. 6, a configuration may also be adopted in which, in addition to the root portion 6ar of the air/water feeding button 6a and the root portion 6br of the suction button 6b, for example, a root portion 7ar of the observation mode switching switch 7a that is another function switch is arranged between the first intersection point P1 and the second intersection point P2 that are formed between the outermost diametrical path 5S and the upper-side side face. That is, the number of function switches arranged between the first intersection point P1 and the second intersection point P2 is not limited to two, and may be three or more.

Note that, in the above described embodiment, in a configuration in which two function switches are arrayed on the upper-side side face, the two function switches may also be disposed as described above, and arrayed in parallel with the longitudinal axis of the operation portion 3. However, in the case of a configuration in which three or more function switches are arrayed on the upper-side side face, a configuration may be adopted in which two function switches that are disposed on the grasp-assistance face 35a side are arrayed on a straight line in parallel with the longitudinal axis of the operation portion, and the other function switches are disposed on the aforementioned straight line or are disposed at positions that deviate from the straight line.

Furthermore, in the above described embodiment, the up/down knob 5 is adopted as the bending operation apparatus. However, the bending operation apparatus may also have a configuration in which, in addition to the up/down knob, a left/right knob (not illustrated in the drawings) is rotatably provided in an overlapping manner on the same axis with respect to the up/down knob 5.

It should be understood that the present invention is not limited only to the above described embodiment, and various modifications thereof can be made without departing from the spirit and scope of the invention.

The present application is filed claiming the priority of Japanese Patent Application No. 2013-043303 filed in Japan on March 5, 2013, and the above described disclosure is incorporated by reference in the present description, claims, and drawings.

## Claims

1. An endoscope comprising:
an operation portion including a grasping portion which is grasped by an operator and a grasp-assistance face on which the operator performs operation and a finger of the operator is to be disposed when the operator grasps the grasping portion;
an insertion portion extended from a distal end side of the operation portion, the insertion portion including on a distal end side thereof a bending portion;
at least one bending operation knob that is provided on one side face of the operation portion and that, by a rotational operation centering on a rotation axis, causes the bending portion to bend; and
at least two function switches that are provided so as to protrude from a side face on one side that configures a side in one direction of the one side face of the operation portion, the at least two function switches being disposed along a longitudinal axis of the operation portion, wherein
switch root portions of the function switches are disposed so as to be positioned between two intersection points at which an outermost diametrical path formed by an outermost circumferential portion of the bending operation knob that is projected onto a plane having a normal line along a rotation central axis of the bending operation knob and the side face on one side intersect with each other.

2. The endoscope according to claim 1, wherein:
heights at which the two function switches arrayed on the side face on one side protrude from the side face on one side are identical heights to each other.

3. The endoscope according to claim 1, wherein:
among heights at which the two function switches arrayed on the side face on one side protrude from the side face on one side, a height of a first function switch that is disposed on a side of the grasp-assistance face that configures a side of the insertion portion on the side face on one side is lower than a height of a second function switch that is positioned further on a proximal end side than the first function switch.

4. The endoscope according to claim 1, wherein:
at least two function switches of the function switches are arrayed on a straight line that is parallel with a longitudinal axis of the operation portion.

5. The endoscope according to claim 4, wherein:
three or more function switches are arrayed on the side face on one side,
the first function switch and the second switch that are provided on the side of the grasp-assistance face are arrayed in parallel on the straight line along a longitudinal direction of the operation portion, and other function switches are disposed on the side face on one side at positions that deviate from the straight line.
